# EUROPÄISCHE PATENTSCHRIFT

(11) **EP 1 827 561 B1**
(45) Veröffentlichungstag und Bekanntmachung des Hinweises auf die Patenterteilung: **14.01.2009**
(21) Anmeldenummer: 05815803.1
(22) Anmeldetag: 22.11.2005
(51) Int. Cl.: A61M 35/00, A61B 19/08

(54) **VORRICHTUNG ZUR WUNDBEHANDLUNG**
DEVICE FOR TREATING WOUNDS
DISPOSITIF DE TRAITEMENT DE PLAIES

(30) Priorität: 23.11.2004 DE 102004056456
(43) Veröffentlichungstag der Anmeldung: 05.09.2007
(73) Patentinhaber: Malamutmann, Viktor, 44359 Dortmund (DE); Malamutmann, Eugen, 44359 Dortmund (DE)
(72) Erfinder: Malamutmann, Viktor, 44359 Dortmund (DE); Malamutmann, Eugen, 44359 Dortmund (DE)
(74) Vertreter: TBK-Patent
(86) Internationale Anmeldenummer: PCT/EP2005/012494
(87) Internationale Veröffentlichungsnummer: WO 2006/056408

(56) Entgegenhaltungen:
- EP-A- 0 355 186
- WO-A-91/08793
- WO-A-03/070135
- DE-A1- 10 252 917
- FR-A- 2 789 595
- GB-A- 2 307 180
- GB-A- 2 378 392
- US-A- 2 280 915
- US-A- 4 909 242
- US-A- 5 624 419
- US-A- 5 941 859
- US-A1- 2004 211 431

## Beschreibung

Die Erfindung betrifft eine Vorrichtung zur Wundbehandlung gemäß dem Oberbegriff des Anspruch 1.

Der Einsatz von Behandlungsmedien wie Flüssigkeiten, Gasen oder auch festen Stoffen ist zielführend, um den Heilungsvorgang einer Wunde zu beschleunigen. Um dabei die Wundoberfläche steril zu halten und eine Verschmutzung der Umgebung auszuschließen, ist es allerdings erforderlich, den Wundbereich durch einen Verband oder eine Abdeckung zu schützen. Außerdem ist es nützlich, Zutritt zu der Wundoberfläche zu erlangen, ohne den Verband bzw. die Abdeckung zu entfernen.

Die DE 28 09 828 A1 offenbart eine Vorrichtung zum Ausspülen von Wunden. Diese besteht aus einer mit einem Rand verbundenen Hülle, wobei entweder die Hülle oder der Rand mit einer Einlass- und einer Auslassöffnung für die verwendete Spülflüssigkeit versehen wird.

Zur Applikation von Wirkstoffen ist in der DE 197 22 075 C2 eine Vorrichtung offenbart, bei der eine poröse Einlage auf eine Wunde aufgebracht wird, die von einer Folie dicht abgedeckt ist. Über eine Zuleitung und eine Ableitung wird ein flüssiger Wirkstoff zu- bzw. abgeführt.

In der EP 0 505 478 B1 ist ein Behandlungssystem für Wunden und andere Störungen offenbart. Dieses Behandlungssystem besteht aus einer flexiblen, anpassbaren Kammer mit einer Balgenfalte zur Aufnahme eines Behandlungsmediums, die um den Umfang einer Wunde herum verschließbar ist. Optional enthält das System Zulassmittel zum Einführen und Entfernen von Behandlungsmedien, Steuermittel für Behandlungsvariable und Überwachungsmittel zur Überwachung von Wundzuständen.

Die DE 196 81 649 T1 offenbart eine transportierbare therapeutische Vorrichtung zur Stimulierung der Heilung von oberflächlichen Wunden. Diese Vorrichtung umfasst ein Gehäuse mit einer Saugpumpe und einem Behälter zur Aufnahme von Flüssigkeit, die durch die Pumpe von der Wunde abgesaugt wurde. Außerdem sind Kontrollmittel und Indikatoren an dem Gehäuse angebracht.

Die Druckschrift EP 0 355 186 A1 offenbart eine Vorrichtung zum Befeuchten und Entwässern von Wunden. Um einen Kontakt mit der Wunde zu verhindern, ist ein hohlzylindrischer offener Kragen mittels einer flexiblen Folie an einer Klebefläche befestigt. Die Folie ist an dem Umfang des unteren Endes des Kragens abgedichtet und an der Fläche der Klebefläche angeschweißt oder mittels Klebstoff angebracht. Die Folie weist einen balgartig ausgebildeten Abschnitt aus, der sich an die Konturen des Körpers anpassen kann. Öffnungen zum Befeuchten der Wunde sind in der Wand des Kragens angeordnet. In der flexiblen Folie ist eine Entleerungsöffnung vorgesehen. Das obere Ende des Kragens ist so ausgeführt, dass es mit einer abnehmbaren Abdeckung verschlossen werden kann.

Es ist die Aufgabe der Erfindung eine verbesserte Vorrichtung zur Wundbehandlung zu schaffen, die eine intensivierte Wundversorgung ermöglicht.

Die Aufgabe der Erfindung wird durch eine Vorrichtung zur Wundbehandlung nach Anspruch 1 gelöst. Vorteilhafte Ausführungsformen werden gemäß den abhängigen Ansprüchen ausgeführt.

Eine erfindungsgemäße Vorrichtung zur Wundbehandlung hat eine Abdeckung zur Bildung einer Kammer über der Wunde, an der mindestens eine in die Kammer ragende Funktionseinrichtung zur Behandlung der Wunde angebracht ist.

Diese Funktionseinrichtung besteht mindestens aus einer Manipulationseinrichtung zum Behandeln der Wunde.

Die Kammer ist durch mindestens ein an der Haut anbringbares Unterteil und mindestens ein Oberteil ausgebildet. Dabei ist das Oberteil vom Unterteil abnehmbar ausgebildet. Zwischen der Haut und dem Unterteil liegt eine Hautschutzfolie. Mindestens eine Manipulationseinrichtung der Funktionseinrichtung ist als Elektrode ausgeführt und in dem Oberteil ist eine mit einem Luftfilter versehene Luftzufuhr- bzw. Luftabfuhr. öffnung vorgesehen.

Gemäß einem Gesichtspunkt der Erfindung besteht die Manipulationseinrichtung aus einer Zufuhreinrichtung zum Zuführen von Behandlungsmedien und einer Abfuhreinrichtung, um vorhandene Medien und Wundsekret wieder aus dem Mundbereich abzuführen. Insbesondere sind die Zufuhreinrichtung und die Abfuhreinrichtung als Zufuhr- bzw. Abfuhrschläuche ausgeführt. Diese Ausführung ermöglicht neben der gleichmäßigen Behandlung der gesamten Wundoberfläche eine gezielte Behandlung einzelner Wundstellen.

Dabei ist es möglich, mehrkanalige Schläuche einzusetzen, so dass durch einen Schlauch die Zufuhr von mindestens zwei verschiedenen Behandlungsmedien erfolgen kann. Außerdem ist durch einen weiteren Kanal auch die Abfuhr des Mediums von der Wunde möglich. Als Behandlungsmedium dienen sowohl flüssige, als auch gasförmige oder feste Stoffe, wobei eine beliebige Kombination dieser Stoffe möglich ist.

Ebenso ist es möglich, andere Einrichtungen wie beispielsweise eine chirurgische Eingreifeinrichtung als Manipulationseinrichtung einzusetzen, um zum Beispiel Abstriche von der Wundoberfläche zu nehmen, oder um abgestorbenes Gewebe zu entfernen.

Insbesondere können auch weitere Elektrode, Thermoelemente, UV-Bestrahlungselemente oder Laserstrahlelemente als Manipulationseinrichtung vorgesehen sein.

Zusätzlich kann die Funktionseinrichtung eine die Funktion der Manipulationseinrichtung unterstützende Hilfseinrichtung aufweisen.

Gemäß einem Gesichtspunkt ist die Hilfseinrichtung als Positionierungselement ausgebildet. Mit Hilfe von diesem Positionierungselement ist es möglich, die Manipulationseinrichtung an einer bestimmten Position innerhalb der Kammer zu halten.

Die Positionierungselemente können ein in der Abdeckung drehbar abgestütztes Bedienelement aufweisen, das einen exzentrischen, in die Kammer ragenden Abschnitt hat. Dieser exzentrische Abschnitt ist jeweils mit einem in der Kammer befindlichen Abschnitt der Manipulationseinrichtung in Eingriff, wobei der entsprechende Abschnitt der Manipulationseinrichtung dadurch beliebig positionierbar ist.

Zusätzlich kann das Positionierungselement aus einem plastischen Werkstoff hergestellt sein. Durch eine Vielzahl von Positionierungselementen wird es somit möglich, entsprechende Abschnitte der Manipulationseinrichtungen genau an bestimmten Positionen innerhalb der Kammer zu anzuordnen. Die Positionierungselemente dienen dabei insbesondere dazu, die Zufuhr- bzw. Abfuhreinrichtung so in der Kammer anzuordnen, dass eine gezielte Behandlung eines ausgewählten Bereichs der Wundoberfläche möglich ist.

Erfindungsgemäß wird die Kammer mittels einem dauerhaft an der Haut eines Patienten anbringbaren Unterteil und mindestens einem abnehmbaren Oberteil über der Wunde gebildet. Das Unterteil ist bevorzugt aus einem weichen Material ausgeführt, das sich an die Form eines Körpers anpasst. Durch die geteilte Ausführung der derart ausgebildeten Kammer ist zu jeder Zeit ein direkter Zugriff auf die Wundoberfläche möglich, falls dies erforderlich ist.

Im zusammengebauten Zustand ist das Innere der Kammer fluiddicht gegen die Umgebung abgedichtet. Das Unterteil kann insbesondere durch Annähen, durch Kleben oder durch sonstige geeignete Verfahren an der die Wunde umgebenden, gesunden Haut angebracht werden.

Bevorzugt befindet sich zwischen dem Unterteil und der gesunden Haut ein Folienelement. Dieses Folienelement wird an die Form der Wunde angepasst, um einerseits die gesunde Haut vor den Behandlungsmedien zu schützen und andererseits als Dichtelement zu dienen.

In dem Oberteil ist eine Vielzahl von fluiddicht verschließbaren Durchtritten vorgesehen, durch die eine Vielzahl an unterschiedlichen Funktionseinrichtungen geführt werden können.

Das Oberteil kann aus einem transparenten Werkstoff ausgeführt sein, wodurch eine visuelle Inspektion des Kammerinneren möglich ist. Dadurch ist es einerseits möglich zu überprüfen, ob die Funktionseinrichtungen korrekt angeordnet sind, und andererseits möglich, die Oberfläche der Wunde selbst visuell zu inspizieren. Zu dem gleichen Zweck kann das Oberteil mit im geschlossenen Zustand fluiddichten Klappen versehen sein.

Gemäß einem Gesichtspunkt der Erfindung ist das Unterteil in Form von mindestens einer aufblasbaren Manschette und das Oberteil aus mindestens zwei Teilen ausgebildet, wobei eine im Wesentlichen zylindrische Kammer entsteht. Durch diese Form der Kammer ist es möglich, Wunden im Bereich von Armen und Beinen wirkungsvoll zu behandeln.

Insbesondere ist die Kammer zur Behandlung von Wunden an Händen und Füßen in Form eines Beutels ausgeführt. Zur Vermeidung von Druckstellen sind Schutzvorrichtungen an entsprechenden Stellen in der Kammer vorgesehen.

Insbesondere ist es auch möglich, eine oben erwähnte Kammer zur Behandlung von Armen und Beinen an einem Ende mit einer oben erwähnten Kammer zur Behandlung von Händen oder Füßen zu verbinden, wobei die Manschette in dem Bereich des Verbindungsabschnitts weggelassen wird. Somit entsteht eine verlängerte Kammer, mit der die Behandlung von sich von dem Fuß oder der Hand entlang des Beins bzw. Arms erstreckenden Wunden möglich ist.

Gemäß einem weiteren Gesichtspunkt ist das Unterteil aus einem Endlosprofil ausgebildet, dessen Enden mit einem Verbindungselement verbunden sind. Das Oberteil wird aus Rippenelementen und einem Folienelement gebildet. In den Rippenteilen ist eine Vielzahl von fluiddicht verschließbaren Öffnungen vorgesehen, durch die die Funktionseinrichtungen in die Kammer geführt werden. Diese Konstruktion ermöglicht ein sehr flexibles Anpassen an die Form der Wunde.

Die Abgabe der Behandlungsfluide erfolgt aus einer Abgabevorrichtung, die als Endelement einer Zufuhreinrichtung ausgeführt ist. Die Abgabevorrichtung weist eine Vielzahl Kammern auf, wobei ein erstes Fluid aus einer ersten Kammer in ein zweites Fluid in einer zweiten Kammer eingespritzt wird. Das dadurch entstehende Fluidgemisch wird dann nach außen abgegeben.

Bevorzugt ist dabei das erste Fluid eine Flüssigkeit und das zweite Fluid ein Gas, wobei die Flüssigkeit in das Gas eingespritzt und zu einem Nebel zerstäubt wird. Der Nebel wird dann durch Abgabeöffnungen aus der zweiten Kammer nach außen zur Wunde abgegeben.

Im Folgenden werden mit Bezug auf die Figuren mehrere Ausführungsbeispiele der Erfindung genau beschrieben.
Fig. 1 zeigt eine aufgeschnittene Ansicht einer an einer Wunde angewendeten Wundreinigungskammer.
Fig. 2 zeigt eine zweite Ausführungsform der Wundreinigungskammer, die insbesondere zur Verwendung im Bereich des Kreuzbeins (Dekubitus) vorgesehen ist.
Fig. 3 zeigt eine aufgeschnittene Ansicht einer Kammer gemäß einer dritten Ausführungsform, die zum Einsatz im Bereich eines Fußes vorgesehen ist.
Fig. 4 zeigt eine aufgeschnittene Ansicht einer Kammer gemäß der dritten Ausführungsform, die zum Einsatz an einem Arm oder einem Bein vorgesehen ist.
Fig. 5 zeigt eine aufgeschnittene Ansicht einer Kammer gemäß der dritten Ausführungsform, die zum Einsatz im Bereich einer Hand vorgesehen ist.
Fig. 6 zeigt eine aufgeschnittene Ansicht einer Kammer gemäß einer vierten Ausführungsform, die zur punktuellen Anwendung bei einer Fistel vorgesehen ist.
Fig. 7 zeigt eine aufgeschnittene Ansicht einer Kammer einer nicht unter der Erfindung zu verstehende Ausführungsform.

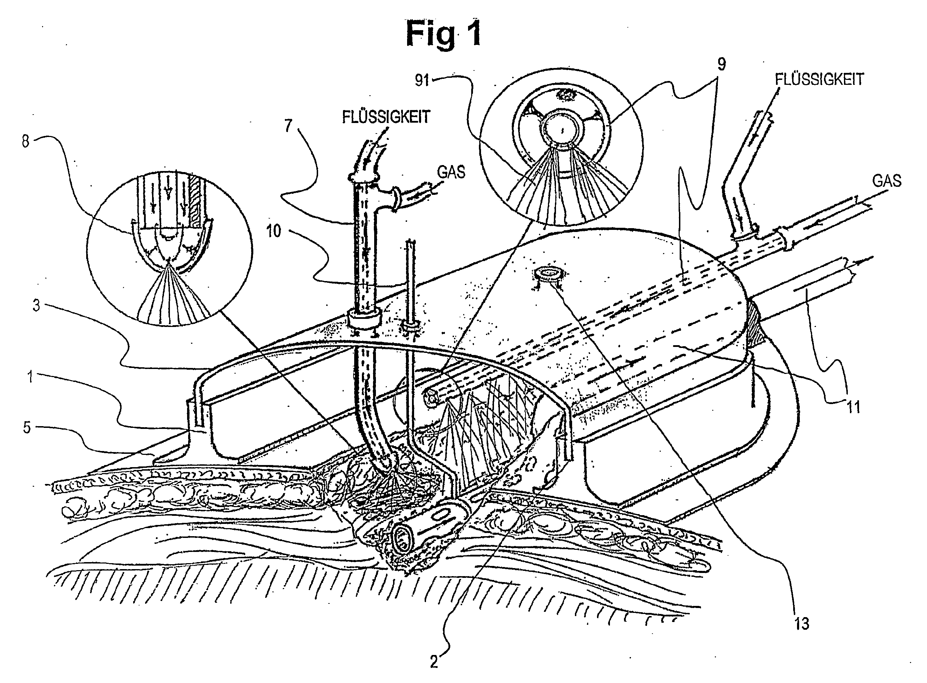

Der grundlegende Aufbau einer erfindungsgemäßen Wundbehandlungsvorrichtung ist aus Fig. 1 ersichtlich. Ein aus einem weichen Werkstoff hergestelltes Unterteil 1 besteht aus einem Wandabschnitt und einem unter dem Wandabschnitt angeordneten Befestigungsabschnitt und bildet mit einem Oberteil 3 eine Kammer. Das Unterteil 1 ist im Bereich einer Wunde an der gesunden Haut des Patienten befestigt. Zum Schutz der gesunden Haut ist zwischen dieser und der Unterseite des Unterteils 1 eine an die Form der Wunde angepasste Hautschutzfolie 5 vorgesehen. Die Hautschutzfolie 5 dient zum Schutz der gesunden Haut innerhalb der Kammer vor den Behandlungsmedien. Dazu wird die Hautschutzfolie 5 mittels einer geeigneten Vorrichtung wie z.B. einer Schere an die Form der Wunde angepasst. Die Befestigung des Unterteils erfolgt durch nicht gezeigte Nähte. Durch den weichen Werkstoff kann sich das Unterteil optimal an eine beliebige Körperform anpassen.

Das Oberteil 3 wird in eine an der Oberseite des Unterteils 1 vorgesehene Nut 2 eingeschoben. In dem Oberteil sind Öffnungen 13 vorgesehen, durch die mindestes ein als Manipulationseinrichtung dienender Zufuhrschlauch 7, 9 in das Innere der Kammer geführt wird.

Gemäß der vorliegenden Ausführungsform ist der Zufuhrschlauch 9 als zweilumiger Schlauch ausgeführt, durch den die gleichzeitige Zufuhr von zwei unterschiedlichen Medien z.B. die Zufuhr einer Flüssigkeit und eines Gases ermöglicht wird. Das Versprühen der Behandlungsmedien erfolgt im Allgemeinen durch in dem Schlauch vorgesehene Abgabeöffnungen 91, die in gleichmäßigen Abständen in einem Umfangs- und Längsabschnitt des Schlauchs angeordnet sind.

Für eine punktuelle Behandlung einzelner Wundstellen können die Behandlungsmedien auch durch eine am Ende eines Zufuhrschlauchs 7 vorgesehene Düse 8 zugeführt werden. Die Öffnungen 13 dienen als Manipulationsöffnungen zum Einbringen von weiteren Manipulationseinrichtungen in die Kammer oder zur Luftzufuhr bzw. -Abfuhr. Zum Schutz der Wunde einerseits und der Umgebung andererseits ist in den zur Luftzufuhr bzw. -Abfuhr vorgesehenen Öffnungen ein bekannter Luftfilter bereitgestellt.

Durch eine im Unterteil 1 vorgesehene Öffnung ist ein ebenfalls als Manipulationseinrichtung dienender Abfuhrschlauch 11 als Abfuhreinrichtung für die Behandlungsmedien und Wundsekret geführt. Der Abfuhrschlauch 11 wird durch mindestens ein durch eine Öffnung im Oberteil 3 in das Innere der Kammer geführtes Positionierungsteil 10 in Position gehalten, wobei das Positionierungsteil 10 einen exzentrischen Abschnitt aufweist und von der Außenseite der Kammer drehbar ist. Dadurch ist es möglich, für den Abfuhrschlauch 11 innerhalb der Kammer eine beliebige Position einzustellen. Das Positionierungsteil 10 erfüllt dabei die Funktion als Hilfseinrichtung und dient gemeinsam mit den Zu- und Abfuhrschläuchen 7, 9 als Funktionseinrichtung.

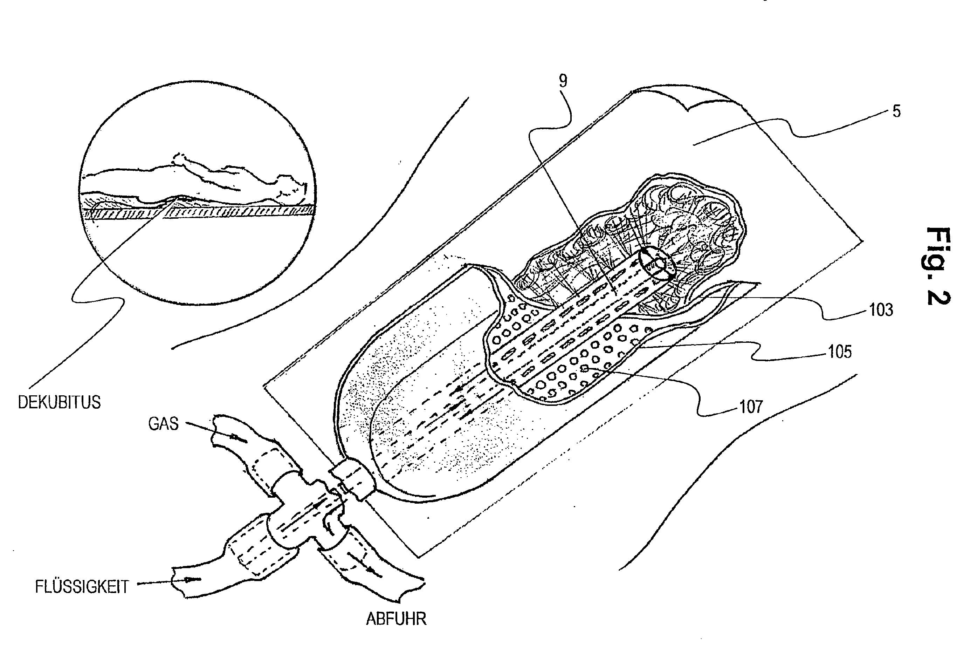

Fig. 2 zeigt eine zweite Ausführungsform, die insbesondere zur Behandlung von Wunden im Rückenbereich vorgesehen ist. Eine innere Wand 103 eines doppelwandig ausgeführten Oberteils ist mit einem integrierten Zufuhrschlauch/ Abfuhrschlauch 9 versehen.

Um ein Ansammeln der abzuführenden Fluide im Wundbereich zu vermeiden, ist die innere Wand 103 des Oberteils mit Öffnungen 107 versehen, durch die das Fluid in den Bereich zwischen den beiden Wänden 103 und 105 treten kann. Aus diesem Bereich wird es durch die dritte Kammer des Schlauchs 9 abgeführt.

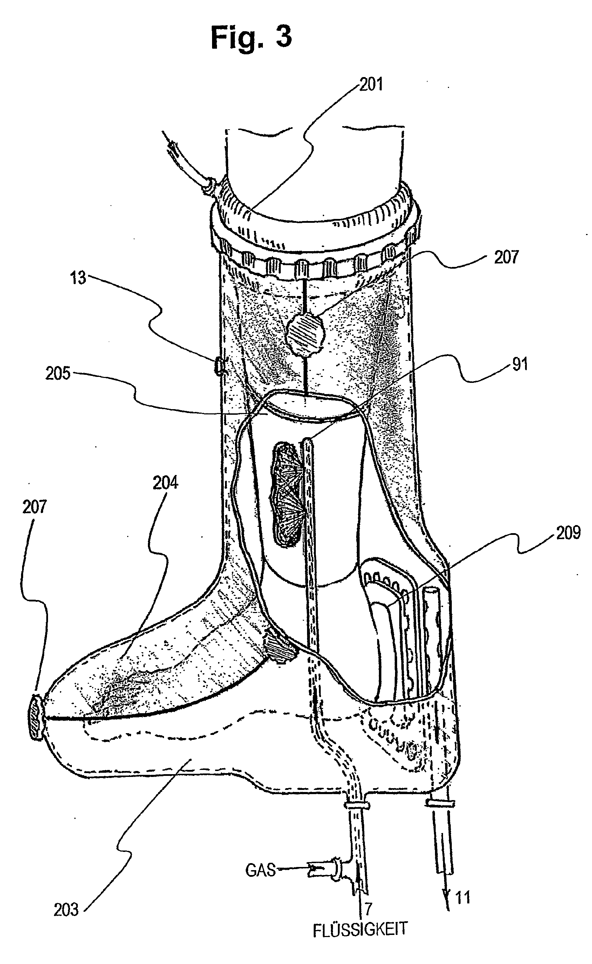

Fig. 3 zeigt eine dritte Ausführungsform der Erfindung, die zum Einsatz bei Wunden im Fußbereich vorgesehen ist. Bei dieser Ausführungsform ist das Unterteil als aufblasbare Manschette 201 ausgeführt, um den gesamten Umfang des Beins abzudichten. Das Oberteil 3 dieser Ausführungsform besteht aus zwei Teilen 203 und 204, die miteinander verbunden werden und dabei gemeinsam mit der Manschette 201 eine der Form des Fußes angepasste Kammer bilden.

Zur Befestigung der Zufuhrschläuche 7 dienen Hilfseinrichtungen 207, die außerdem die Teile 203, 204 entlang deren Verbindungslinie zusammenhalten. Als Zufuhrvorrichtung 7 ist wieder ein Zufuhrschlauch 7 eingesetzt. Zur Abfuhr der jeweiligen Medien dient der im Fersenbereich vorgesehene Abfuhrschlauch 11.

Zur Vermeidung von Druckstellen sind Gelkissen 209 als Schutzvorrichtung in den für Druckstellen gefährdeten Bereichen vorgesehen. Außerdem ist zum Schutz der gesunden Haut auch hier eine Hautschutzfolie angewendet.

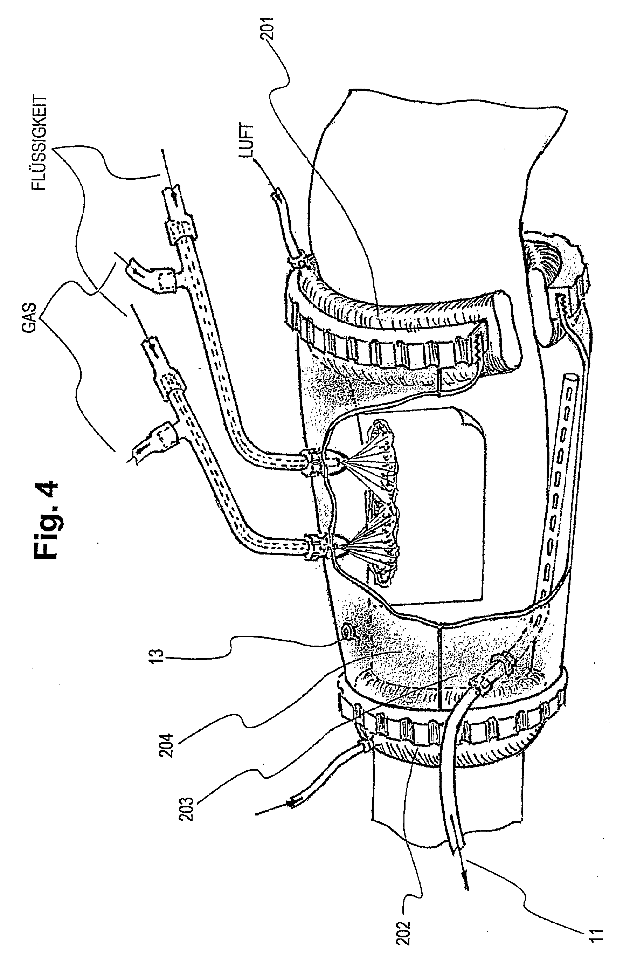

In Fig. 4 ist eine Anwendung der dritten Ausführungsform bei einer Wunde im Bereich der Beine oder Arme gezeigt. Als Unterteil werden am oberen Ende und am unteren Ende der Kammer jeweils eine Manschette 201, 202 eingesetzt. Die Zufuhr und Abfuhr der jeweiligen Medien erfolgt entsprechend den Erfordernissen zur Behandlung der Wunde durch die bereits erläuterten Zufuhr- und Abfuhrvorrichtungen. Das Oberteil dieser Kammer besteht aus zwei Teilen 203 und 204.

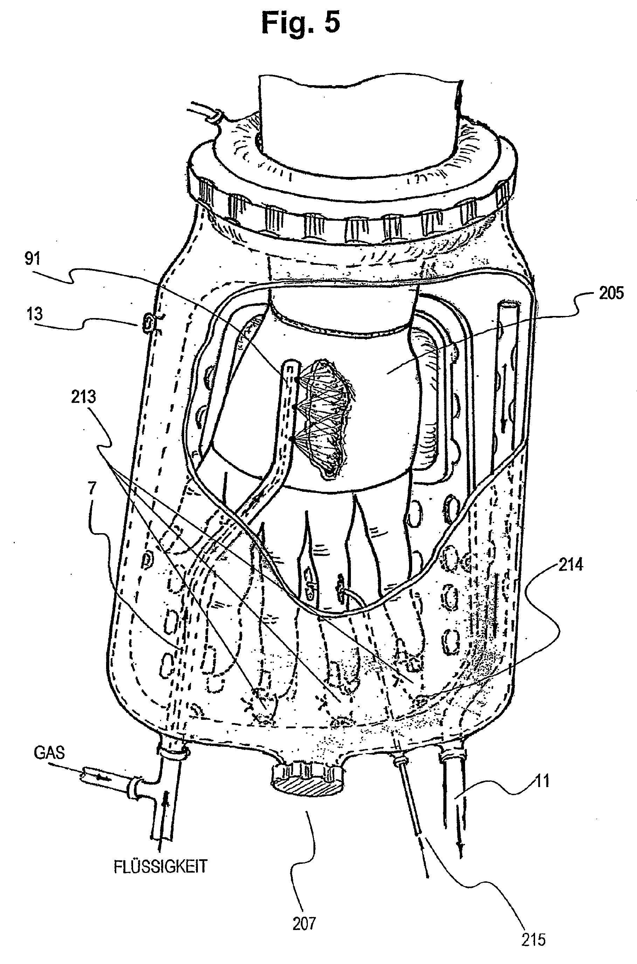

Fig. 5 zeigt die Anwendung der dritten Ausführungsform bei einer Wunde im Handbereich. Zur Fixierung der Finger sind Fixierungsösen 214 und Fixierungspunkte 213 im Bereich der Fingerspitzen vorgesehen. Im Übrigen entspricht die in Fig. 5 gezeigte Kammer mit Ausnahme von Größe und Form der in Fig. 3 gezeigten Kammer.

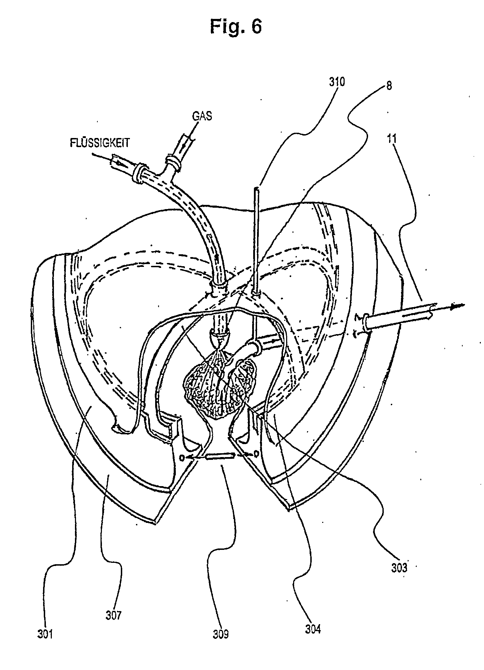

Fig. 6 zeigt eine vierte Ausführungsform der vorliegenden Erfindung. Bei dieser Ausführungsform ist das Unterteil aus einem Endlosprofil 301 ausgebildet, dessen Enden mit einem Verbindungselement 309 verbunden werden. Das Endlosprofil 301 wird mit einer dazwischen vorgesehenen Hautschutzfolie 307 an der Haut befestigt. Ein die Abfuhrvorrichtung bildender Abfuhrschlauch 11 ist durch eine Öffnung im Endlosprofil 301 geführt.

Das Oberteil gemäß dieser Ausführungsform wird aus Rippenelementen 303 und einer Abdeckfolie 304 ausgebildet. Nach der Befestigung des Endlosprofils 301 auf der Haut wird das aus Rippenelementen 303 bestehende Gerüst des Oberteils gebildet, wobei die Enden der Rippenelemente 303 in einer Nut an der Oberseite des Endlosprofils 301 angeordnet werden. In jedem Rippenelement sind Öffnungen vorgesehen, die denen der ersten Ausführungsform entsprechen. Die Wundreinigungskammer gemäß dieser Ausführungsform kann den gesamten Körper abdecken. Dies ist insbesondere bei der Behandlung von großflächigen Verbrennungen vorteilhaft.

Nach dem Anbringen der anhand der ersten Ausführungsform bereits erläuterten Zufuhr- 7, Abfuhr- 11 und Fixiervorrichtungen 310 wird das Oberteil durch das Anbringen einer Abdeckfolie 304 fertiggestellt. Gemäß der vorliegenden Ausführungsform ist die Folie 304 durch Kleben mit dem Endlosprofil 301 verbunden. Nach dem Ankleben der Folie erfüllt die Kammer die gleichen Funktionen wie die Kammer der ersten Ausführungsform.

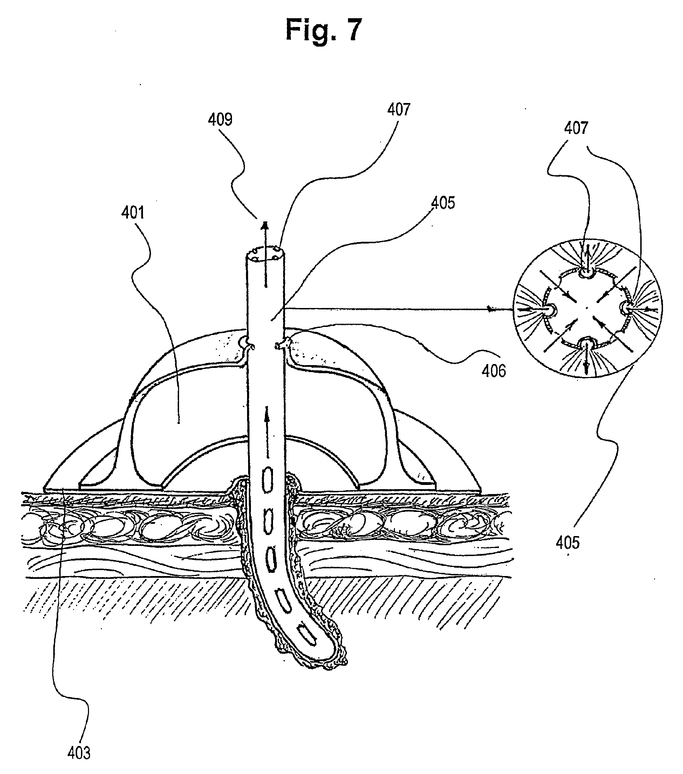

Eine nicht unter der Erfindung zu verstehende Ausführungsform ist in Fig. 7 gezeigt. Die Kammer gemäß der vierten Ausführungsform dient der Behandlung kleiner Wunden oder Fehlstellen wie z.B. einer Fistel. Gemäß dieser Ausführungsform sind das Unterteil und das Oberteil als kombinierte Abdeckkammer 401 ausgeführt.

Durch eine Öffnung 406 in der Abdeckkammer 401 ist ein kombinierter Zufuhr-/ Abfuhrschlauch 405 geführt. Das Ende dieses Schlauchs 405 ist bis zum Ende der Fistel in diese eingeführt. Danach wird die Abdeckkammer 401 an der Haut befestigt, wie bereits bei der ersten Ausführungsform beschrieben wurde. Durch in dem Schlauch 405 vorgesehene Spülkanäle 407 wird ein Behandlungsmedium zu der Fistel zugeführt, das dann zusammen mit Wundsekret durch einen Abfuhrkanal 409 wieder abgeführt wird.

Mit sämtlichen Ausführungsformen der Erfindung ist ein Steuergerät kombinierbar. Mit diesem Steuergerät ist das Programmieren der Fluidzufuhr und -abfuhr, der Menge der Behandlungsmedien und das Einstellen des Innendrucks der Kammer möglich. Insbesondere kann mit dem digitalen Steuergerät auch das Gas- und Flüssigkeitsvolumen und das Absaugen der Flüssigkeiten geregelt werden.

## Patentansprüche

1. Vorrichtung zur Wundbehandlung mit einer Abdeckung zur Bildung einer Kammer über der Wunde, an der mindestens eine in die Kammer ragende Funktionseinrichtung (5, 7, 8, 9, 10, 11, 13, 115, 210, 305) bestehend aus mindestens einer Manipulationseinrichtung zur Behandlung der Wunde angebracht ist, wobei die Kammer durch mindestens ein an der Haut anbringbares Unterteil (1, 101, 201) und mindestens ein Oberteil (3; 103, 104; 203, 204) ausgebildet ist, und das Oberteil vom Unterteil abnehmbar ausgebildet ist, und eine Hautschutzfolie (5) vorgesehen ist, zwischen der Haut und dem Unterteil (1, 101, 201) zu liegen,
**dadurch gekennzeichnet, dass**
mindestens eine Manipulationseinrichtung (5, 7, 8, 9, 11, 13, 115, 305) der Funktionseinrichtung als Elektrode (5) ausgeführt ist, und dass in dem Oberteil (3; 103, 104; 203, 204) eine mit einem Luftfilter versehene Luftzufuhr- bzw. Abfuhröffnung (13) vorgesehen ist.

2. Vorrichtung nach Anspruch 1, **dadurch gekennzeichnet, dass** eine weitere Manipulationseinrichtung als chirurgische Eingriffseinrichtung und/oder als Zufuhreinrichtung (7, 8, 9, 13, 115, 305) und Abfuhreinrichtung (11) ausgebildet ist.

3. Vorrichtung nach Anspruch 2, **dadurch gekennzeichnet, dass** die Zufuhreinrichtung (7, 8, 9, 13, 115, 305) und die Abfuhreinrichtung (11) als Zufuhr- bzw. Abfuhrschläuche zum Zuführen eines Mediums in die Kammer bzw. Abführen eines Mediums aus der Kammer ausgebildet sind.

4. Vorrichtung nach Anspruch 3, **dadurch gekennzeichnet, dass** die Zufuhr- bzw. Abfuhrschläuche mehrkanalig ausgebildet sind.

5. Vorrichtung nach Anspruch 4, **dadurch gekennzeichnet, dass** die Zufuhr- und Abfuhrschläuche in einem Strang einteilig ausgebildet sind.

6. Vorrichtung nach einem der Ansprüche 1 bis 5, **dadurch gekennzeichnet, dass** die Funktionseinrichtung zusätzlich mindestens eine Hilfseinrichtung (10, 210) aufweist.

7. Vorrichtung nach Anspruch 6, **dadurch gekennzeichnet, dass** die Hilfseinrichtung als Positionierungselement (10, 210) zur Positionierung der Manipulationseinrichtung (7, 8, 9, 11, 13, 115, 305) ausgebildet ist.

8. Vorrichtung nach Anspruch 7, **dadurch gekennzeichnet, dass** das Positionierungselement (10) ein an der Abdeckung drehbar abgestütztes Bedienelement mit einem in die Kammer ragenden exzentrischen Abschnitt aufweist.

9. Vorrichtung nach Anspruch 7 oder 8, **dadurch gekennzeichnet, dass** das Positionierungselement (10, 210) aus einem plastischen Werkstoff ausgebildet ist.

10. Vorrichtung nach einem der Ansprüche 1 bis 9, **dadurch gekennzeichnet, dass** das Unterteil (1, 101, 201) aus mindestens einer Manschette (101) besteht, und das Oberteil (103, 104) im Wesentlichen zylinderförmig und mindestens zweiteilig ausgebildet ist.

11. Vorrichtung nach Anspruch 10, **dadurch gekennzeichnet, dass** das Oberteil (103, 104) in Form eines Beutels ausgebildet ist, an dessen Öffnung die Manschette (101) angeordnet ist.

12. Vorrichtung nach Anspruch 10, **dadurch gekennzeichnet, dass** das Oberteil (103, 104) in Form eines offenen Zylinders ausgeführt ist, an dessen beiden Enden jeweils eine Manschette (101, 102) angeordnet ist.

13. Vorrichtung nach einem der Ansprüche 10 bis 12, **dadurch gekennzeichnet, dass** die Manschette (101, 102) aufblasbar ausgeführt ist.

14. Vorrichtung nach einem der Ansprüche 1 bis 9, wobei das Unterteil aus einem endlosen Profil (201) hergestellt ist, und das Oberteil aus einem auf Versteifungselementen (203) abgestützten Folienelement (204) besteht.

15. Vorrichtung nach einem der Ansprüche 1 bis 9, wobei das Oberteil mit einer Innenwand (103) und einer Außenwand (105) doppelwandig ausgeführt ist, und die Innenwand (103) Öffnungen (107) aufweist.

## Claims

1. A device for the treatment of wounds, comprising a cover for forming a chamber above a wound, at which at least one functional means (5, 7, 8, 9, 10, 11, 13, 115, 210, 305) protruding into the chamber and including at least one manipulating means is arranged for treating the wound, wherein the chamber is formed by at least one lower part (1, 101, 201) attachable to the skin and at least one upper part (3; 103, 104; 203, 204), and the upper part is designed to be detachable from the lower part, and a skin protective film (5) is provided to be disposed between the skin and the lower part (1, 101, 201),
**characterized in that**
at least one manipulating means (5, 7, 8, 9, 11, 13, 115, 305) of the functional means is designed as an electrode (5), and **in that** an air supply and discharge opening (13) provided with an air filter is provided in the upper part (3; 103, 104; 203, 204).

2. A device according to claim 1, **characterized in that** a further manipulating means is formed as a surgical intervention means and/or a supply means (7, 8, 9, 13, 115, 305) and discharge means (11).

3. A device according to claim 2, **characterized in that** the supply means (7, 8, 9, 13, 115, 305) and the discharge means (11) are formed as supply and/or discharge tubes for supplying a medium into the chamber and/or discharging a medium from the chamber.

4. A device according to claim 3, **characterized in that** the supply and/or discharge tubes are multi-channel tubes.

5. A device according to claim 4, **characterized in that** the supply and discharge tubes are formed in a line in one piece.

6. A device according to any one of claims 1 to 5, **characterized in that** the functional means additionally includes at least one auxiliary means (10, 210).

7. A device according to claim 6, **characterized in that** the auxiliary means is formed as a positioning element (10, 210) for positioning the manipulating means (7, 8, 9, 11, 13, 115, 305).

8. A device according to claim 7, **characterized in that** the positioning element (10) has an operating element rotatably supported on the cover and comprising an eccentric portion protruding into the chamber.

9. A device according to claim 7 or 8, **characterized in that** the positioning element (10, 210) is made of a plastic material.

10. A device according to any one of claims 1 to 9, **characterized in that** the lower part (1, 101, 201) consists of at least one sleeve (101), and the upper part (103, 104) is substantially cylindrical and is formed of at least two pieces.

11. A device according to claim 10, **characterized in that** the upper part (103, 104) is in the form of a bag at the opening of which the sleeve (101) is disposed.

12. A device according to claim 10, **characterized in that** the upper part (103, 104) has the shape of an open cylinder at the two ends of which a sleeve (101, 102) is arranged, respectively.

13. A device according to any one of claims 10 to 12, **characterized in that** the sleeve (101, 102) is designed so as to be inflatable.

14. A device according to any one of claims 1 to 9, wherein the lower part is made of a continuous profile (201), and the upper part consists of a film element (204) supported on reinforcing elements (203).

15. A device according to any one of claims 1 to 9, wherein the upper part is double-walled, comprising an inner wall (103) and an outer wall (105), and the inner wall (103) has openings (107).

## Revendications

1. Dispositif pour le traitement de plaies, avec un recouvrement pour la formation d'une chambre au-dessus de la plaie, sur laquelle est monté au moins un dispositif fonctionnel (5, 7, 8, 9, 10, 11, 13, 115, 210, 305), composé d'au moins un dispositif de manipulation, pénétrant dans la chambre, pour le traitement de la plaie, la chambre étant réalisée au moyen d'au moins une partie inférieure (1, 101, 201) susceptible d'être appliquée sur la peau, et d'au moins une partie supérieure (3 ; 103, 104 ; 203, 204), et la partie supérieure étant réalisée de manière à pouvoir être retirée de la partie inférieure, et une feuille de protection de la peau (5) est prévue, se plaçant entre la peau et la partie inférieure (1, 101, 201),
**caractérisé en ce qu'**
au moins un dispositif de manipulation (5, 7, 8, 9, 11, 13, 115, 305) du dispositif fonctionnel est réalisé sous forme d'électrode (5), et **en ce que**, dans la partie supérieure (3 ; 103, 104 ; 203, 204), est prévue une ouverture d'amenée d'air ou d'évacuation d'air (13), munie d'un filtre à air.

2. Dispositif selon la revendication 1, **caractérisé en ce qu'**un autre dispositif de manipulation est réalisé sous forme de dispositif d'intervention chirurgicale et/ou de dispositif d'amenée (7, 8, 9, 13, 115, 305) et dispositif d'évacuation (11).

3. Dispositif selon la revendication 2, **caractérisé en ce que** le dispositif d'amenée (7, 8, 9, 13, 115, 305) et le dispositif d'évacuation (11) sont réalisés sous forme de tuyaux d'amenée ou d'évacuation, pour l'amenée d'un fluide dans la chambre ou d'évacuation d'un fluide hors de la chambre.

4. Dispositif selon la revendication 3, **caractérisé en ce que** les tuyaux d'amenée ou d'évacuation sont à plusieurs canaux.

5. Dispositif selon la revendication 4, **caractérisé en ce que** les tuyaux d'amenée ou d'évacuation sont réalisés d'une seule pièce, en un brin.

6. Dispositif selon l'une des revendications 1 à 5,
**caractérisé en ce que** le dispositif de manipulation présente en plus au moins un dispositif auxiliaire (10, 210).

7. Dispositif selon la revendication 6, **caractérisé en ce que** le dispositif auxiliaire est réalisé sous forme de dispositif de positionnement (10, 210), pour assurer le positionnement du dispositif de manipulation (5, 7, 8, 9, 11, 13, 115, 305).

8. Dispositif selon la revendication 7, **caractérisé en ce que** le dispositif de positionnement (10) présente un élément de manoeuvre, prenant appui à rotation sur le recouvrement, avec un tronçon excentrique, pénétrant dans la chambre.

9. Dispositif selon la revendication 7 ou 8, **caractérisé en ce que** le dispositif de positionnement (10, 210) est réalisé en une matière synthétique plastique.

10. Dispositif selon l'une des revendications 1 à 9,
**caractérisé en ce que** la partie inférieure (1, 101, 201) est composée d'au moins une manchette (101), et la partie supérieure (103, 104) est réalisée sensiblement en forme de cylindre et au moins en deux parties.

11. Dispositif selon la revendication 10, **caractérisé en ce que** la partie supérieure (103, 104) est réalisée sous la forme d'un sachet, à l'ouverture duquel est disposée la manchette (101).

12. Dispositif selon la revendication 10, **caractérisé en ce que** la partie supérieure (103, 104) est réalisée sous la forme d'un cylindre ouvert, à chacune des deux extrémités duquel est disposée une manchette (101, 102).

13. Dispositif selon l'une des revendications 10 à 12, **caractérisé en ce que** la manchette (101, 102) est gonflable.

14. Dispositif selon l'une des revendications 1 à 9,
**caractérisé en ce que** la partie inférieure est fabriquée à partir d'un profil (201) sans fin, et la partie supérieure est composée d'un élément en feuille (204) soutenu sur des éléments de rigidification (203).

15. Dispositif selon l'une des revendications 1 à 9,
**caractérisé en ce que** la partie supérieure est réalisée à paroi double, avec une paroi intérieure (103) et une paroi extérieure (105), et la paroi intérieure (103) présente des ouvertures (107).
